# EUROPEAN PATENT APPLICATION

(11) **EP 2 620 761 A1**
(43) Date of publication of application: **31.07.2013**
(21) Application number: 13152748.3
(22) Date of filing: 25.01.2013
(51) Int. Cl.: G01N 21/35, G01N 33/14, G01N 21/90

(54) **Group for the in-line measuring of the quantity of carbon dioxide dissolved in a liquid contained in a closed container**

(30) Priority: 30.01.2012 IT MI20120108
(71) Applicant: Ft System S.r.l., 29010 Alseno (PC) (IT); L Pro S.r.l., 35143 Padova (PD) (IT)
(72) Inventor: Forestelli, Fabio, 29010 CASTELNUOVO FOGLIANI (PC) (IT); Tondello, Paolo, 35010 LIMENA (PD) (IT)
(74) Representative: Lotti, Giorgio

(57) **Abstract**

The present invention refers to a group for the in-line measuring of the quantity of carbon dioxide dissolved in a liquid contained in a closed container such as bottles, jars and so on and to an automatic containers filling plant comprising such measuring group. The measuring group according to the present invention comprises at least one inspection area (20,20') suitable for housing at least one container (11), at the at least one inspection area (20,20') being provided at least one laser spectroscopy device (16) for measuring the partial pressure of carbon dioxide present in the head space (13) of a container (11) placed in the at least one inspection area (20,20'), and it is characterised in that at the at least one inspection area (20,20') there are additionally provided means for mixing (12) the content of said container (11).

## Description

The present invention refers to a group for the in-line measuring of the quantity of carbon dioxide dissolved in a liquid contained in a closed container such as bottles, jars and so on and to an automatic containers filling plant comprising such measuring group.

In the field of automatic filling of containers made of various types of materials, such as plastic material, glass and so on, there are known filling plants usually made up of a station for filling the containers, followed by a station for closing and/or capping the containers, as well as one or more control stations placed downstream of the closing station.

The filling stations and the closing stations in turn comprise, respectively, a plurality of taps or filling valves and closing and/or capping heads of the mechanical or electronic type depending on the particular design of the plant.

The filling valves and the closing/capping heads are initially calibrated or set electronically so as to obtain - in output - the desired result in terms of filling and closing, depending on the particular container intended to be treated.

The actual obtainment of the setpoint filling and closing parameters is subsequently monitored by the possible control stations placed downstream, which allow to inspect the filled bottles or containers, determining whether the same present or not the filling and closing characteristics intended to be obtained. The measuring groups used in the relative control stations currently arranged in line allow determining some parameters including the filling level and pressure within a bottle or container.

However, there are other parameters that may provide valid information as regards a malfunctioning process, for example regarding the step of closing/capping the container.

In case of liquids added with carbon dioxide, such as non-alcoholic drinks or beer, it is for example interesting to detect the quantity of carbon dioxide dissolved in the liquid.

Actually, such measuring provides an indication regarding the correct bottling process in terms of filling and/or capping the bottle/container or, however, an indication regarding a correct processing of the liquid prior to bottling thereof.

For example, in case of improper capping, in particular, in case of lack of sealing, the carbon dioxide contained is no longer in equilibrium between the liquid and gaseous phase. The carbon dioxide component dissolved in the liquid tends to assume the gaseous state, thus reducing progressively.

In order to retrace the amount of the carbon dioxide dissolved in the liquid within a closed container, it is currently known to perform sample tests that provide for collecting a given quantity of the content of the previously filled and closed container, and measuring, for example through chemical analysis, the quantity of carbon dioxide dissolved and present in the same.

Besides requiring the presence of an operator for managing the steps of picking up the container from the line and the disposal thereof after the analysis, this type of test is of the destructive type, no longer being able to recover the examined container.

For this reason, as of date it is not possible to measure all the processed containers directly in line, but offline sample test alone.

Thus, it is difficult to retrace a systematic error for example caused by a given filling valve or a capping head. Actually, a sample test provides a probability indication as regards the presence of a process criticality, but without offering definite information regarding the source of a possible detected malfunctioning.

In order to measure the quantity of carbon dioxide dissolved in a liquid contained in containers at least partly made of optically transparent material, in particular plastic material or glass, there is known the possibility of using laboratory instruments of the so-called spectroscopy laser type, i.e. instruments that detect the absorbtion of a laser beam of a suitable wavelength projected in the head space of the closed container, i.e. in the area comprised between the closure and free surface of the liquid present in the container.

This allows determining the concentration or partial pressure of the carbon dioxide present in gaseous form in the head space of the container, which, in conditions of equilibrium, is proportional to the quantity of carbon dioxide dissolved in the liquid present in the container.

Thus, such instrument performs a non-invasive type of measuring, maintaining the condition of the container intact. However, with the aim of a correct measuring, there is required the intervention of an operator who - besides managing the picking up of the container from the line and the possible re-introduction into the line should the measuring lead to acceptable results - should evaluate whether the quantity of carbon dioxide dissolved in the liquid and that present in the gaseous phase in the head space are in equilibrium with respect to each other before proceeding to the measuring operations.

Actually, after filling and capping the container, the carbon dioxide contained therein is in a non-equilibrium condition which does not allow performing a correct measuring.

At the temperature conditions at which a filling plant usually operates equivalent to about 10-18°C, the condition of equilibrium between the quantity of carbon dioxide dissolved in the liquid and that present in the head space is spontaneously achieved over a period of time of about 3 hours. Such times are incompatible with the need of performing a measuring operation directly in line.

It is however possible to catalyze the achievement of the equilibrium conditions through a suitable handling of the container, usually carried out by the operator, thus reducing the standby times before performing the useful measuring operation.

Last but not least, the laboratory instruments known to date for non-destructive measuring of the quantity of carbon dioxide dissolved in a liquid contained in a closed container simply provide the detected value, which should be subsequently interpreted by the operator with the aim of evaluating whether to discard the measured container or re-introduce it in the line, as well as the causes that may have led to an abnormal measuring value.

In conclusion, the instruments known up to date require continuous intervention by an operator, thus not being suitable for use in a measuring station placed in an automatic filling plant.

An object of the present invention is to overcome the aforementioned drawbacks and in particular that of creating a group for measuring the quantity of carbon dioxide dissolved in a liquid contained in closed containers that do not require any intervention by an operator, thus being suitable for use in an automatic filling plant.

Specifically, an object of the present invention is to provide a group for measuring the quantity of carbon dioxide dissolved in a liquid contained in closed containers that is capable of operating a non-destructive measuring and in quick times, thus allowing to perform the control on a large sample of containers. Another object of the present invention is to provide a group for measuring the quantity of carbon dioxide dissolved in a liquid contained in closed containers that is capable of automatically accelerating the achievement of conditions of equilibrium of the carbon dioxide contained in the container.

A further object of the present invention is to provide an automatic containers filling plant capable of carrying out the systematic measuring of the quantity of carbon dioxide dissolved in the liquid.

Last but not least an object of the present invention is to conceive an automatic containers filling plant capable of automatically managing the interpretation of the measuring of the quantity of carbon dioxide dissolved in the liquid in terms of discarding the containers deemed faulty, determining the mechanical parts causing the defect and/or calibrating the same. These and other objects according to the present invention are attained by providing a group for measuring the quantity of carbon dioxide dissolved in a liquid contained in closed containers as outlined in claim 1.

Further characteristics of the measuring group are subject of the dependent claims 2-7.

Such objects are also obtained with an automatic containers filling plant as outlined in claim 8. Further characteristics of the automatic filling plant are indicated in the dependent claims 9-16. Characteristics and advantages of a group for measuring the quantity of carbon dioxide dissolved in a liquid contained in closed containers according to the present invention shall be clearer from the following exemplifying and non-limiting description with reference to the attached schematic drawings wherein:
- figure 1 is a schematic representation - in plan view
- of a group for measuring the quantity of carbon dioxide dissolved in a liquid contained in closed containers according to the present invention;
- figure 1a is a schematic representation - in front elevational view - of a first detail of the measuring group of figure 1;
- figure 1b is a schematic representation - in front elevational view - of a second detail of the measuring group of figure 1;
- figure 2 is a schematic representation - in plan view
- of an automatic filling plant comprising a group for measuring the quantity of carbon dioxide dissolved according to the present invention.

With reference to the figures, a group for measuring the quantity of carbon dioxide dissolved in a liquid contained in a closed container 11 is shown indicated in its entirety with 10.

The group 10 for measuring the quantity of dissolved carbon dioxide comprises at least one inspection area 20,20' suitable for receiving and housing at least one container 11.

A laser spectroscopy device 16 for measuring the partial pressure of carbon dioxide present in the head space 13 of a container 11 placed in the at least one inspection area 20,20' is provided at the at least one inspection area 20,20'.

It is assumed that the container 11 subject of the measuring is made of optically transparent material at least at a portion of the head space 13 thereof.

The device 16 for spectroscopy laser measuring comprises at least one laser source 17, preferably adjustable, capable of emitting a laser beam 18 at a given wavelength, wherein the laser source 17 is positioned so that the emitted laser beam 18 traverses the at least one inspection area 20' suitable for housing a container 11.

In particular, the laser source 17 is positioned so as to emit the laser beam 18 towards the inspection area 20,20' at the head space 13 of the container 11, and more in particular, at the portion of container 11 made of optically transparent material.

The expression head space of a closed container 11 it is meant to indicate the volume within the closed container 11 interposed between the closure 14 and the free surface of the liquid content 15 of the container 11.

The emission wavelength of the laser beam 18 is preferably adjusted so as to substantially coincide with the wavelength for absorbing the gas of interest. In the present case in which it is of interest to determine the concentration or partial pressure of the carbon dioxide, the wavelength for adjusting the laser beam is preferably comprised in the range between 1.8 - 2.2 µm (microns).

The device 16 for spectroscopy laser measuring also comprises a detector 19 facing the laser source for detecting the laser beam 18' attenuated following the absorption occurred at the head space 13 of the container 11 placed in the inspection area 20,20'.

The detector 19 is also such to provide - in output - an item representative of the spectrum for the absorption of the carbon dioxide present in the head space 13 of the container 11 on the basis of the detected attenuated laser beam 18'.

According to the present invention, at the at least one measuring area 20,20' there are provided means for mixing 12 the content 15 of the container made up of a heterogeneous system comprising at least one liquid and carbon dioxide dissolved in the same.

The mixing means 12 are such to act on a container 11 placed in the inspection area 20,20' with the aim of stimulating the process of reaching an equilibrium between the carbon dioxide dissolved in the liquid contained in the closed container 11 and the carbon dioxide present in gaseous phase in the head space 13 of the same 11.

The mixing means 12 are preferably a mixing device of the mechanical type.

Alternative embodiments selected as a function of the particular type of container and treated filling liquid, provide for the possibility of using - additionally or alternatively to the mechanical mixer 12 - any kind of device capable of inducing a mixing of the heterogeneous liquid-carbon dioxide system 15 present in the container at molecular level.

By way of example, there may be used a ultrasonic mixer and/or a heat transfer device and/or a microwave device or a combination of devices of the aforementioned types.

For the sake of illustrative clarity, the figures show an embodiment of the measuring group 10 according to the present invention in which the device 16 for spectroscopy laser measuring acts on a container 11 placed in a first successive inspection area 20' with respect to a second inspection area 20 at which the mixing means 12 act.

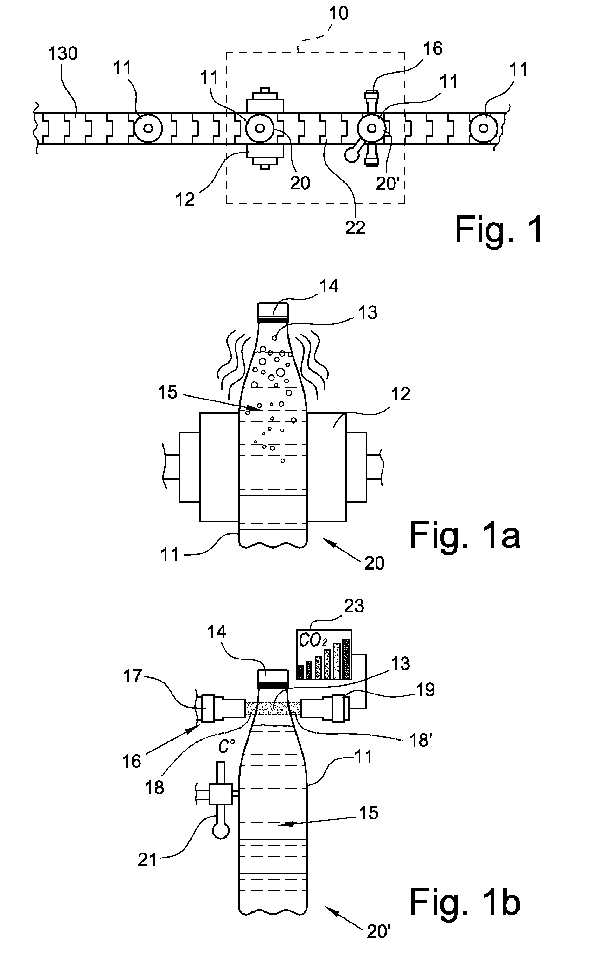

In such a configuration, the measuring group 10 comprises automatic conveying means 22, represented in a schematic and entirely exemplifying manner in figure 1 through a conveyor belt, for the automatic displacement of the container between the first 20 and the second 20' inspection area.

In an alternative embodiment (not illustrated) the mixing means 12 and the device 16 for spectroscopy laser measuring act in succession at a same inspection area 20,20'. Thus, such embodiment does not comprise automatic conveying means 22.

The measuring group 10 preferably comprises electronic processing means 23 suitable for processing the data output by the detector 19 of the device 16 for spectroscopy laser measuring with the aim of determining the quantity of carbon dioxide dissolved in the liquid present in the analysed container 11 and comparing the obtained results with acceptability ranges for determining the suitability of such container 11.

On the basis of the processed data it is optionally possible to automatically control the discarding of the container 11.

Preferably, there are additionally provided temperature detecting means 21 of a container 11 present in the at least one inspection area 20,20'.

The temperature detecting means 21 preferably act simultaneously on the device 16 for spectroscopy laser measuring and they are connected to the electronic processing means 23 with the aim of correlating the measuring value of the partial pressure of carbon dioxide present in the head space 13 of the container 11 with the temperature of the liquid contained in the same 11.

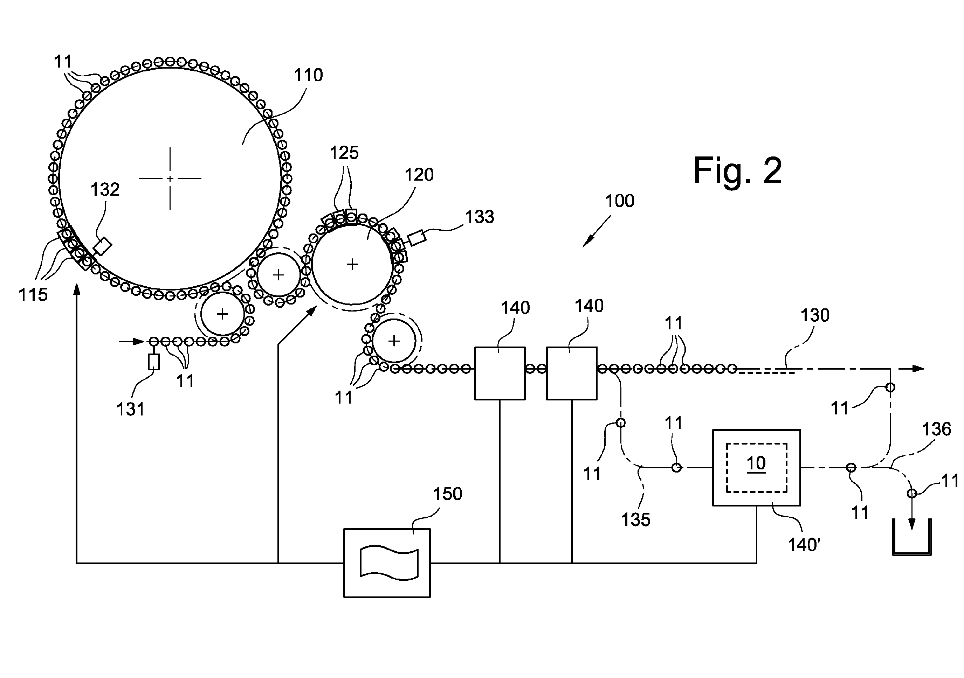

The group 10 for measuring the quantity of carbon dioxide dissolved in a liquid contained in a closed container 11 is preferably comprised in an automatic filling plant indicated in its entirety with 100 and illustrated by way of example in figure 2.

Such plant 100 comprises a first station 110 for filling containers 11, followed by a second station 120 for closing and/or capping the containers 11.

The filling 110 and closing 120 stations in turn comprise a plurality, respectively, of taps or filling valves 115 and closing and/or capping heads 125 constrained to move forward along the peripheral of the respective first and second station 110,120 so as to follow the containers 11 under treatment over a stretch, thus performing the filling and/or capping thereof in movement.

Preferably, the first 110 and the second 120 station are circularly shaped, wherein the taps or filling valves 115 and the closing and/or capping heads 125 are constrained to the peripheral of a rotary joust or carousel. Such stations 110,120 may for example be tool holders respectively provided with about eighty taps or filling valves 115 and about twenty closing and/or capping heads 125.

The containers 11 are conveyed through special conveying means 130, such as for example a set of conveying means constrained or free on a conveyor belt or suspended, along a forward movement route which at least partly follows the periphery of the first 110 and the second 120 station.

The forward movement route travelled by the containers 11 is divided into a plurality of discrete positions assumed by the containers 11 during the forward movement along the filling plant 100.

Downstream of the second station 120 with respect to the direction of forward movement of the container 11 there is also provided at least one control station 140.

The control stations 140 may be arranged in line along the main forward movement route of the containers 11, or along a secondary parallel branch 135 of such route, depending on whether a test is being carried out on all the filled containers 11 or a sample test solely on a subset of the containers 11 treated and conveyed along the secondary branch 135 by special deviating means (not illustrated).

The control stations 140 may for example comprise one or more from among the following measuring modules:
- a module for measuring the filling level,
- a module for measuring the tension of the side walls,
- a module for measuring the capping height,
- a module for measuring the removal torque and reclosing angle,
- a module for measuring the gas content and/or pressure,
- a module for measuring the weight, or
- a module for measuring the colour.

In particular, the plant 100 for filling containers 11 comprises at least one control station 140' provided with a group 10 for measuring the quantity of carbon dioxide dissolved in a liquid according to the present invention.

Preferably, the control station 140' provided with a group 10 for measuring the quantity of carbon dioxide dissolved in a liquid is placed along the secondary parallel branch 135 of the forward movement route.

In order to pass and stop in the at least one inspection area 20,20' of the measuring group 10 the containers 11 are picked up from the main route of forward movement through the deviating means and conveyed towards such inspection area 20,20' through the secondary branch 135 of the conveying means 130 of the plant 100.

There are also provided means (not illustrated) for discarding containers 11 identified as faulty or unsuitable by the group 10 for measuring the quantity of carbon dioxide dissolved in a liquid, which convey the faulty containers along a branch 136 for discarding the conveying means 130.

The discarding means are controlled by the electronic processing means 23 of the measuring group 10. Furthermore, there are provided means (not illustrated) for re-introducing the containers 11 identified as suitable in the main route of forward movement of the conveying means 130 also controlled by the electronic processing means 23 of the measuring group 10.

Both the discarding means and the re-inserting means are placed downstream of the measuring group 10 with respect to the direction of forward movement of the containers 11.

According to a preferred embodiment, entering the conveying means 130 there is provided a first position sensor 131 which detects the passage of a first container 11.

Analogously, to a first filling tap 115 and a first capping head 125 there are preferably associated respective position sensors 132,133 which keep track of the position thereof 115,125 within the respective stations 110,120.

In such preferred embodiment there is also provided a special encoder (not illustrated) associated with the conveying means 130 which, known the relative distances in terms of pitch-machine, keeps track of the positions-machine in which the containers 11, the taps 115 and the capping heads 125 are located.

This allows to retrace the particular tools (tap 115 and capping head 125) acting on each container 11.

The single control stations 140,140' are connected to an electronic processing unit 150 for processing control data detected and associated with a particular capping head 125 and/or filling tap 115 due to the positions-machine data provided by the encoder.

In particular, the electronic processing unit 150 recognises which tap 115 filled the container 11 and which capping head 125 closed the same 11 and, on the basis of such data, it creates a database of the received measuring values, associated to a particular capping head 125 and/or tap 115, determining a plurality of statistic parameters, such as the average value, the standard deviation or statistic indices indicating the capacity of the system to produce a result within predefined limits.

This allows to define the statistical behaviour of each capping head 125 and/or tap 115, offering the possibility of analysing the trend of statistical parameters within a time range with the aim of detecting a variation of such values over time due to wear or drift and quantify the degree thereof.

For example, a variation of the measuring of the dissolved carbon dioxide over time may be correlated to a progressive reduction of the sealing of the closure of the container due to a progressively less efficient capping, due to a progressive malfunctioning or wear of the relative capping head 125.

Alternatively, a variation of the measuring of the dissolved carbon dioxide over time may be correlated to a progressive loss of sealing between the gasket of a filling tap and the opening of the container to be filled. In such circumstances, there actually occurs a pressure difference between the filling vat and the container itself, hence, an effervescence of the liquid during the filling leading to the dispersion of part of the originally dissolved carbon dioxide.

In a preferred embodiment, the electronic processing means 23 of the measuring group 10 are possibly integrated in the unit 150 for processing the filling plant 100.

The processing unit 150 is also preferably connected to means (not illustrated) for adjusting the first and second station 110,120 which control the operating parameters thereof with the aim of performing a calibration on the basis of the detected variations. For example, in plants in which the carbon dioxide is directly added in line, on the basis of the detected data it is possible to adjust the operating parameters of the first station 110 with the aim of obtaining the desired quantity of dissolved carbon dioxide.

This allows performing a continuous and automatic calibration of the single stations 110,120 whose correct operation is automatically and precisely determined on the basis of variations in the relative statistical behaviour.

The group 10 for measuring the quantity of carbon dioxide dissolved in a liquid contained in a closed container 11 operates as follows.

A container 11 placed in the measuring area 20 is initially subjected to mechanical and/or thermal and/or electromagnetic and/or acoustic mixing with the aim of bringing the carbon dioxide dissolved in the liquid contained in the closed container 11 and the carbon dioxide present in gaseous phase in the head space 13 of the same 11 to an equilibrium condition. Subsequently, the partial pressure or concentration of carbon dioxide comprised in the head space 13 of the container is measured.

For such a purpose, the laser source 17 emits, towards the measuring area 20' suitable for receiving a container 11, a laser beam 18 at a wavelength that can be set in a range of a few nanometres and comprised in the range of 1.8 - 2.2 µm (microns).

In particular, the laser beam 18 is emitted towards the measuring area 20' at the height of the head space 13 of the container 11, and precisely, at the container portion 11 made of optically transparent material.

The detector 19 detects the laser beam 18' attenuated following absorption occurred at the head space 13 of the container 11 and provides in output a data representative of the spectrum of absorption of the carbon dioxide present in the head space 13.

A special processing of the data provided output by the detector 19 through the processing means 23 allows to determine the concentration of carbon dioxide comprised in the head space 13 and, on the basis of the same, the quantity of carbon dioxide dissolved in the liquid.

In conditions of equilibrium, the quantity of carbon dioxide dissolved in the liquid is proportionally related to the concentration of carbon dioxide comprised in the head space 13 with a known proportionality factor, different according to the analysed liquid.

On the basis of the obtained measuring data, there occurs the evaluation of the suitability of the analysed container 11: should the quantity of carbon dioxide dissolved in the liquid subject of detection fall outside a preset acceptation range, the container is classified as unsuitable and discarded subsequently. Otherwise, should the values of carbon dioxide dissolved in the liquid subject of detection be within the acceptation range, the container is deemed suitable, thus being suitable to be re-introduced into the line.

Preferably, for a more accurate evaluation of the suitability of the analysed container 11 there occurs the detection of the temperature of the container 11. The acceptation range of the container is thus modified according to the detected temperature.

Should the group 10 for measuring the quantity of carbon dioxide dissolved in a liquid be comprised in an automatic filling plant 100 provided with position sensors 131,132,133 and encoder, the obtained measuring values are associated to a particular tap or filling valve 115 and to a particular closing and/or capping head 125 through the positions-machine provided by the encoder.

Upon collecting a considerable statistic sample of measuring values of the quantity of carbon dioxide dissolved in the liquid of the containers 11 all treated by the same tap or filling valve 115 or by a particular closing and/or capping head 125 the statistical data of such tap 115 and capping head 125 are calculated.

The statistical data are updated upon every new measuring operation however keeping track of the trend of the same over time, with the aim of detecting a variation in the operation of the single taps 115 or capping heads 125 such to require a calibration to bring back to the initially set value of dissolved carbon dioxide.

The description outlined above clearly reveals the characteristics and advantages of the measuring group subject of the present invention.

Actually, the group for measuring the quantity of carbon dioxide dissolved in a liquid contained in closed containers according to the present invention is capable operating in full automation, also providing for special automatic means for attaining the equilibrium condition.

Furthermore, the group for measuring the quantity of carbon dioxide dissolved in a liquid according to the present invention is capable carrying out measuring operations without altering the condition of the examined container, thus making it useable for controlling a large sample of containers.

Last but not least, the automatic containers filling plant according to the present invention is capable of automatically managing picking up a sample from the line, measuring the quantity of carbon dioxide dissolved in the liquid of the examined sample, carrying out the interpretation of the measured values of the quantity of carbon dioxide dissolved in the liquid in terms of suitability of the sample and the possible discarding of the samples deemed faulty.

Last but not least, in preferred embodiments, the automatic containers filling plant according to the present invention is capable of determining the mechanical parts causing the defect and/or calibrate the same.

Lastly, it is clear that the measuring group thus conceived is susceptible to numerous modifications and variants, all falling within the scope of the invention; furthermore, all details can be replaced by technically equivalent elements. In practice the materials used, as well as the dimensions, may vary depending on the technical requirements.

## Claims

1. Group (10) for measuring the quantity of carbon dioxide dissolved in a liquid contained in a closed container (11) made of a material optically transparent at least at one portion of a head space (13) of the same (11), the measuring group (10) comprising at least one inspection area (20,20') suitable for housing at least one container (11), at said at least one inspection area (20,20') at least one laser spectroscopy device (16) being provided for measuring the partial pressure of the carbon dioxide present in the head space (13) of a container (11) placed in said at least one inspection area (20,20'), **characterized in that** means for mixing (12) the content of said container (11) are in addition provided at said at least one inspection area (20,20').

2. Group (10) for measuring the quantity of carbon dioxide dissolved in a liquid contained in a closed container (11) according to claim 1 **characterized in that** said mixing means (12) comprise at least one of
- a mechanical mixer,
- an ultrasonic mixer,
- a heat transfer device and/or
- a microwave device.

3. Group (10) for measuring the quantity of carbon dioxide dissolved in a liquid contained in a closed container (11) according to claim 1 or 2 **characterized in that** said laser spectroscopy measuring device (16) comprises at least a laser source (17) capable of emitting a laser beam (18,18') towards said at least one inspection area (20,20') at the head space (13) of a container (11) placed in the same (20,20'), and at least a detector (19) facing said at least a laser source (17) to detect said laser beam (18,18').

4. Group (10) for measuring the quantity of carbon dioxide dissolved in a liquid contained in a closed container (11) according to any of the preceding claims **characterized in that** it comprises means (21) for detecting the temperature of a container (11) placed in said at least one inspection area (20,20').

5. Group (10) for measuring the quantity of carbon dioxide dissolved in a liquid contained in a closed container (11) according to any of the preceding claims **characterized in that** it comprises electronic processing means (23) suitable for processing measuring data output by said laser spectroscopy device (16).

6. Group (10) for measuring the quantity of carbon dioxide dissolved in a liquid contained in a closed container (11) according to claim 5 when depending on claim 4 **characterized in that** said electronic processing means (23) receive an input measuring data from said temperature detecting means (21) and are suitable for processing said measuring data output by said laser spectroscopy device (16) by putting it in relation with said measuring data of said temperature detecting means (21).

7. Group (10) for measuring the quantity of carbon dioxide dissolved in a liquid contained in a closed container (11) according to any of the preceding claims **characterized in that** it comprises a first inspection area (20) at which said means for mixing (12) the content of said container (11) are provided, a second inspection area (20') at which said at least one laser spectroscopy device (16) is provided, and automatic conveying means (22) for moving a container (11) between said first (20) and said second (20') inspection area.

8. Automatic containers (11) filling plant (100) comprising conveying means (130) through which a plurality of containers (11) is moved along a main direction of forward movement, along said main direction of forward movement there being arranged in succession a first station (110) for filling said containers (11) comprising a plurality of taps or filling valves (115) and a second station (120) for closing and/or capping said containers (11) comprising a plurality of closing and/or capping heads (125), downstream of said second closing and/or capping station (120) there being arranged at least one control station (140,140') for checking at least one process parameter **characterized in that** said at least one control station (140') comprises a group (10) for measuring the quantity of carbon dioxide dissolved in a liquid contained in a closed container (11) according to any of the claims 1 to 7.

9. Automatic containers (11) filling plant (100) according to claim 8 **characterized in that** said at least one control station (140') comprises a group (10) for measuring the quantity of carbon dioxide dissolved in a liquid placed along a secondary branch (135) of said conveying means (130) placed outside of said main direction of forward movement, there being provided deviating means for picking up a subset of containers (11) from said main direction of forward movement and for directing it along said secondary branch (135).

10. Automatic containers (11) filling plant (100) according to claim 8 or 9 **characterized in that** it comprises discarding means placed downstream of said at least one control station (140') comprising a group (10) for measuring the quantity of carbon dioxide dissolved in a liquid, said discarding means being suitable for directing containers (11) identified as defective by said measuring group (10) along a discarding branch (136) of the conveying means (130), said discarding means being controlled by said electronic processing means (23) of said measuring group (10).

11. Automatic containers (11) filling plant (100) according to any of the claims 8 to 10 **characterized in that**, downstream of said at least a control station (140') comprising a group (10) for measuring the quantity of carbon dioxide dissolved in a liquid, it comprises means for re-inserting containers (11) identified as suitable by said measuring group (10) in said main route of forward movement of said conveying means (130), said re-inserting means being controlled by said electronic processing means (23) of said measuring group (10).

12. Automatic containers (11) filling plant (100) according to any of the claims 8 to 11 **characterized in that** said conveying means (130) comprise a plurality of discreet positions that can be assumed by said containers (11), and **in that** it comprises a first position sensor (131) suitable for detecting the passage of a first container (11), a second position sensor (132) associated with a first tap or filling valve (115), a third position sensor (133) associated with a first closing and/or capping head (125) and an encoder associated with said discreet conveying means (130) so as to keep track of the instantaneous positions of said first container (11), of said first tap or filling valve (115) and of said first closing and/or capping head (125).

13. Automatic containers (11) filling plant (100) according to any of the claims 8 to 12 **characterized in that** it comprises an electronic processing unit (150) connected in input to said at least one control station (140,140') of at least one process parameter, to said first (131), second (132) and third (133) position sensors and to said encoder in order to put in relation a detected process parameter relating to a container (11) to a tap or filling valve (115) and a closing and/or capping head (125) having acted on said container (11).

14. Automatic containers (11) filling plant (100) according to claim 13 **characterized in that** said processing unit (150) is connected to adjusting means of said first and second stations (110,120) suitable for controlling the operating parameters of said tap or filling valve (115) and/or of said closing and/or capping head (125) acting on said container (11), on the basis of said detected process parameter.

15. Automatic containers (11) filling plant (100) according to claim 13 or 14 **characterized in that** said electronic processing means (23) of said measuring group (10) are embedded in said electronic processing unit (150).

16. Automatic containers (11) filling plant (100) according to any of the claims 8 to 15 **characterized in that** said at least one control station (140,140') of said at least one process parameter comprises at least one of the measuring modules belonging to the group made of:
- a module for measuring the filling level,
- a module for measuring the tension of the side walls,
- a module for measuring the capping height,
- a module for measuring the removal torque and the reclosing angle,
- a module for measuring the gas content and/or pressure,
- a module for measuring the weight, or
- a module for measuring the colour.
